# EUROPÄISCHE PATENTSCHRIFT

(11) **EP 3 551 142 B1**
(45) Veröffentlichungstag und Bekanntmachung des Hinweises auf die Patenterteilung: **19.05.2021**
(21) Anmeldenummer: 17811541.6
(22) Anmeldetag: 05.12.2017
(51) Int. Cl.: A61F 5/01

(54) **ORTHESE, INSBESONDERE GONARTHROSEORTHESE**
ORTHESIS, SPECIALLY GONARTHROSIS ORTHESIS
ORTHESE, SPECIALEMENT ORTHESE POUR LE TRAITEMENT D'UNE GONARTHROSE

(30) Priorität: 07.12.2016 DE 202016106817 U
(43) Veröffentlichungstag der Anmeldung: 16.10.2019
(60) Teilanmeldung: 20162508.4
(73) Patentinhaber: Thuasne Deutschland GmbH, 30938 Burgwedel (DE)
(72) Erfinder: SCHÖBEL, Herbert, 30938 Burgwedel (DE); VIOLEAU, Nicolas, 42131 La-Valla-en-Gier (FR); PANNETIER, Romain, 69003 Lyon (FR)
(74) Vertreter: Horak, Michael
(86) Internationale Anmeldenummer: PCT/EP2017/081549
(87) Internationale Veröffentlichungsnummer: WO 2018/104316

(56) Entgegenhaltungen:
- WO-A1-94/00082
- WO-A1-98/36713
- WO-A2-2012/006590
- JP-A- 2008 264 151
- US-A- 4 425 912
- US-A- 4 805 606
- US-A- 4 854 308
- US-B1- 6 893 410

## Beschreibung

### Kurzdarstellung der Erfindung

Die Erfindung betrifft eine Orthese, insbesondere Gonarthrose-Orthese zur Entlastung des medialen oder lateralen Kniekompartiments des menschlichen Knies.
WO 98/36713 A1 stellt den nächsten Stand der Technik dar.

Orthetische Hilfsmittel unterstützen die Funktion von Gelenken und werden zur Stabilisierung, Ruhigstellung und/oder Entlastung des entsprechenden Gelenks eingesetzt, um beispielsweise bei Erkrankungen oder Verletzungen, diese zu stabilisieren und zu führen, wodurch eine richtige und schmerzfreie Position eingestellt und gefestigt wird.

Eine gattungsgemäße Orthese ist aus EP 2 246 014 B1 bekannt. Diese Orthese weist ein Gurtsystem mit zwei verstellbaren Gurten auf, die an einer flexiblen Manschette befestigt und über Kreuz angeordnet sind. Mit dieser Orthese kann eine Entlastung des Kniegelenks bei Kniebeschwerden erreicht werden. Die Gurte sorgen dafür, dass sich beim Öffnen des Kniegelenks ein Gelenksspalt öffnet, sodass je nach Anordnung der Orthese entweder das mediale Kniekompartiment oder das laterale Kniekompartiment therapiewirksam entlastet wird und dadurch eine schmerzfreie Bewegung ermöglicht wird.

Mittels der Gurte sollen Kräfte durch einen Druckpunkt lateral oder medial möglichst beinmittig in Höhe der Kompromissdrehachse des Knies aufgebracht werden. Allerdings kann es vorkommen, dass der Druckpunkt tatsächlich zu weit dorsal oder distal, also eher im Kniekehlenbereich, angeordnet ist. Für eine ausreichende Entlastung des Knies müssen durch eine solche fehlerhafte Anordnung hohe Kräfte mithilfe der Gurte aufgebracht werden. Der Kniekehlenbereich ist druckempfindlich, daher kann es in der Folge der fehlerhaften Anordnung und der hohen Zugkraft der Gurte zu Schmerzen beim Tragen der Orthese kommen. Außerdem kann die Beugung des Knies behindert werden. Insgesamt kann der Tragekomfort der in EP 2 246 014 B1 beschriebenen Orthese verringert sein.

Der Erfindung liegt daher die Aufgabe zugrunde, ein orthetisches Hilfsmittel, insbesondere Gonarthrose-Orthese zur Verfügung zu stellen die einen verbesserten Tragekomfort zu den Orthesen im Stand der Technik aufweist.

Die vorliegende Erfindung betrifft daher eine Orthese zur Entlastung des medialen oder lateralen Kniekompartiments des menschlichen Knies nach Anspruch 1, mit einem Gurtsystem, wobei das Gurtsystem wenigstens eine zugstabile Gurtvorrichtung aufweist. Jede Gurtvorrichtung umfasst dabei jeweils wenigstens einen Endgurt und mindestens einen Verbindungsgurt, der winkelverstellbar mit jedem Endgurt der jeweiligen Gurtvorrichtung verbunden ist. Dadurch können ein Winkel im Verlauf der Gurtvorrichtung eingestellt und ein Druckpunkt positioniert werden. Insgesamt ist somit eine anatomische Gurtführung möglich. In einer bevorzugten Ausführungsform ist die Orthese eine Gonarthrose-Orthese.

In einer Ausführungsform weißt das Gurtsystem eine Kreuzführung für zwei Gurtvorrichtungen auf, bei denen die zwei Gurtvorrichtungen sich im Bereich der Kreuzführung kreuzen und mittels der Kreuzführung zwangsgeführt gehalten werden. Der wenigstens eine Endgurt von wenigstens einer Gurtvorrichtung besteht in einer Ausführungsform jeweils aus genau einem proximalen Endgurt der jeweiligen Gurtvorrichtung und genau einem distalen Endgurt der jeweiligen Gurtvorrichtung. Der Verbindungsgurt der jeweiligen Gurtvorrichtung weist hierbei einen proximalen und einen distalen Endbereich auf. Mithilfe des proximalen Endbereichs ist der jeweilige Verbindungsgurt mit dem proximalen Endgurt der jeweiligen Gurtvorrichtung verbunden. Der distale Endbereich verbindet den jeweiligen Verbindungsgurt mit dem distalen Endgurt der jeweiligen Gurtvorrichtung.

In einer weiteren Ausführungsform ist der wenigstens eine Endgurt von wenigstens einer Gurtvorrichtung mittels einer Klettverbindung mit dem Verbindungsgurt der jeweiligen Gurtvorrichtung winkelverstellbar und lösbar verbunden.

Der Verbindungsgurt der jeweiligen Gurtvorrichtung weist in einer weiteren Ausführungsform ein Gurtelement mit einer Vorderseite und einer Rückseite auf. An jedem Ende weist das Gurtelement auf der Rückseite jeweils ein Hakenband für die Klettverbindung und eine korrespondierend zu diesem Hakenband angeordnete und klappbare Lasche mit einem dem Hakenband zugewandten weiteren Hakenband auf. Die Endgurte weisen beidseitig zumindest abschnittsweise ein Schlaufenband auf, das zwischen den Hakenbändern positionierbar ist. Die Endgurte sind mittels der durch die Hakenbänder und die Schlaufenbänder gegebenen Klettverbindung mit dem Verbindungsgurt winkelverstellbar verbindbar.

In einer Ausführungsform ist wenigstens ein Endgurt von wenigstens einer Gurtvorrichtung mittels einer Schraubverbindung oder mittels einer Nietverbindung mit dem jeweiligen Verbindungsgurt der jeweiligen Gurtvorrichtung winkelverstellbar verbunden. In einer bevorzugten Ausführungsform weist der wenigstens eine Endgurt wenigstens einer Gurtvorrichtung zur Längenverstellung einen unteren Gurtabschnitt und einen auf den unteren Gurtabschnitt umgeschlagenen oberen Gurtabschnitt auf, wobei mit diesen Gurtabschnitten eine längenverstellbare Schlaufe ausgebildet ist.

Die Gurtvorrichtung am oberen Gurtabschnitt des Endgurts weist in einer weiteren Ausführungsform einen Schnellverschluss auf, mittels welchem die Schlaufe lösbar geschlossen werden kann. Der Schnellverschluss kann in einer bevorzugten Ausführungsform einen Magneten aufweisen, der den Schnellverschluss magnetisch in eine Schließposition zieht und in der Schließposition verriegelt.

Ein am oberen Gurtabschnitt angeordnetes Ende des Endgurts ist in einer Ausführungsform der vorliegenden Erfindung mittels einer Klettverbindung in einem zur Längenverstellung der Schlaufe wählbaren Bereich befestigt.

Gemäß Anspruch 1 weist die Orthese eine Manschette auf, an der das Gurtsystem befestigt ist. Die wenigstens eine Gurtvorrichtung besteht dabei aus genau einer ersten Gurtvorrichtung mit einem ersten Verbindungsgurt und genau einer zweiten Gurtvorrichtung mit einem zweiten Verbindungsgurt, wobei beide Gurtvorrichtungen einander kreuzen und jeweils an einem proximalen Bereich und an einem distalen Bereich der Manschette befestigt sind. Das Gurtsystem weist weiterhin Befestigungsvorrichtungen mit Seilen auf, durch welche beide Gurtvorrichtungen an der Manschette befestigt sind. Zusätzlich sind am Gurtsystem Umlenkösen vorgesehen, an welchen die Endgurte der Gurtvorrichtungen gehalten werden, wobei jedes Seil jeder Befestigungsvorrichtung, jeweils durch eine Umlenköse hindurchragt und diese Umlenköse in einem Freiheitsgrad der Translation und zwei Freiheitsgraden der Rotation an der jeweiligen Befestigungsvorrichtung hält. In einer bevorzugten Ausführungsform ist die wenigstens eine Befestigungsvorrichtung vertikal versetzbar an der Manschette befestigt. In einer besonders bevorzugten Ausführungsform weist die wenigstens eine Befestigungsvorrichtung ein Basisteil auf, an welchem jedes Seil der jeweiligen Befestigungsvorrichtung an jeweils zwei vertikal voneinander beabstandeten Stellen gehalten wird und zusammen mit dem Basisteil jeweils eine Schlaufe bildet. Das Basisteil kann in einer Ausführungsform ein Hakenband und die Manschette zumindest abschnittsweise ein Schlaufenband aufweisen. Das Hakenband und das Schlaufenband bilden zusammen eine Klettverbindung, mittels der das Basisteil an der Manschette befestigbar und positionierbar ist.

In einer Ausführungsform sind Stellen am Basisteil angeordnet, an denen ein Seil einer Befestigungsvorrichtung und jeweils paarweise mit den Stellen an denen das andere Seil dieser Befestigungsvorrichtung gehalten ist. In einer bevorzugten Ausführungsform ist jedes Seil wenigstens einer Befestigungsvorrichtung ausgehend von jeweils einer der vertikal voneinander beabstandeten Stellen durch das Basisteil hindurch geführt. Das jede Seil wird dabei in dem Basisteil um einen Winkel zwischen 80° und 100° umgelenkt und zum Spannen der jeweiligen Gurtvorrichtung mit einem Ende des Seils aus dem Basisteil herausgeführt. Die jeweilige Befestigungsvorrichtung weist hierbei ein dem Basisteil zugeordnetes Greifteil auf, welches mit den herausgeführten Enden der Seile der jeweiligen Befestigungsvorrichtung verbunden ist.

Die vorliegende Erfindung wird durch die Ausführungsformen in den Ansprüchen gekennzeichnet und durch die Ausführungen in der folgenden Beschreibung und den Zeichnungen näher beschrieben.

### Beschreibung der Zeichnungen

- Fig. 1:: Darstellung des einen ersten Ausschnitts einer erfindungsgemäßen Orthese mit zwei sich kreuzenden Verbindungsgurten, die jeweils winkelverstellbar mit zwei Endgurten verbunden sind.
- Fig. 2:: Darstellung des einen zweiten Ausschnitts der erfindungsgemäßen Orthese von Fig. 1 mit einer Befestigungsvorrichtung für zwei Endgurte.
- Fig. 3:: Darstellung des einen zweiten Ausschnitts von Fig. 1 einer erfindungsgemäßen Orthese gemäß einem zweiten Ausführungsbeispiels der Erfindung mit einer alternativen Befestigungsvorrichtung für zwei Endgurte.
- Fig. 4:: Darstellung der erfindungsgemäßen Orthese der Fig. 1 und Fig. 2 in einer ersten Ansicht (zur vollständigen proximal-distalen Erstreckung).
- Fig. 5:: Darstellung der erfindungsgemäßen Orthese der Fig. 1, Fig. 2 und Fig. 4 in einer zweiten Ansicht (zur vollständigen proximal-distalen Erstreckung).

### Detaillierte Beschreibung der Erfindung

Die vorliegende Erfindung betrifft ein orthetisches Hilfsmittel, insbesondere Orthese (1), die vorzugsweise zur Behandlung der Gonarthrose verwendet werden kann.

Bei einer Orthese mit einem Gurtsystem (2), bei dem das Gurtsystem (2) wenigstens eine zugstabile Gurtvorrichtung (3, 4) aufweist, wobei jede Gurtvorrichtung (3, 4) jeweils wenigstens einen Endgurt (9, 10, 11, 12) umfasst, ist erfindungsgemäß vorgesehen, dass jede Gurtvorrichtung (3, 4) jeweils mindestens einen Verbindungsgurt (7, 8) aufweist, der winkelverstellbar mit jedem Endgurt (9, 10, 11, 12) der jeweiligen Gurtvorrichtung (3, 4) verbunden ist. In einer bevorzugten Ausführungsform ist genau ein Verbindungsgurt (7, 8) vorgesehen, der winkelverstellbar mit jedem Endgurt (9, 10, 11, 12) der jeweiligen Gurtvorrichtung (3, 4) verbunden ist. Dadurch können ein Winkel im Verlauf der Gurtvorrichtung (3, 4) eingestellt und ein Druckpunkt positioniert werden. Insgesamt ist somit eine anatomische Gurtführung möglich. Etwaigen Schmerzen beim Tragen der Orthese wird dadurch wirksam entgegengewirkt, sodass der Tragekomfort stark gesteigert ist. Bei der Orthese handelt es sich vorzugsweise um eine Gonarthroseorthese zur Entlastung des medialen oder lateralen Kniekompartiments des menschlichen Knies.

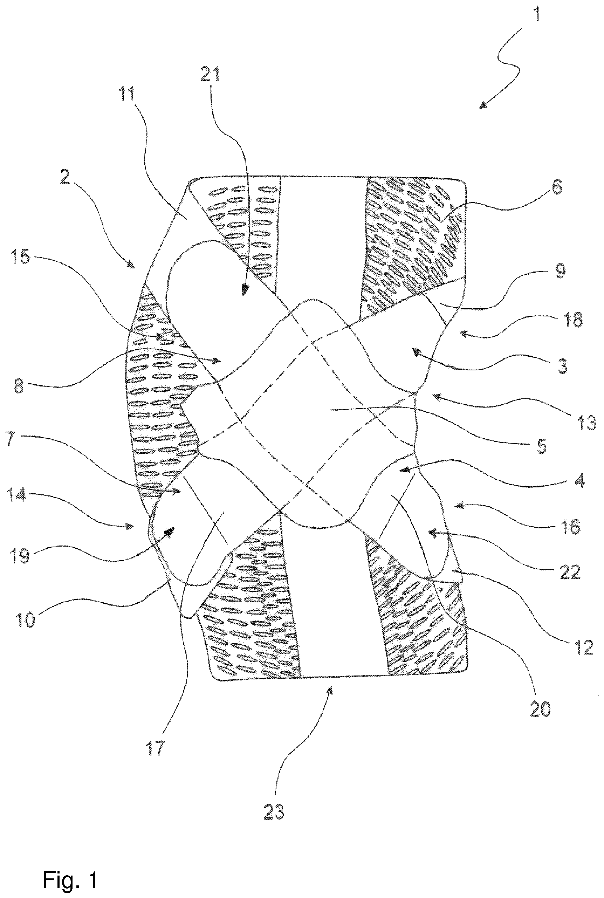

Bei einer Ausführungsform der erfindungsgemäßen Orthese besteht wenigstens eine Gurtvorrichtung (3, 4) aus genau einer ersten Gurtvorrichtung (3) mit einem ersten Verbindungsgurt (7) und genau einer zweiten Gurtvorrichtung (4) mit einem zweiten Verbindungsgurt (8), wie beispielsweise in Fig. 1 dargestellt. Die Orthese weist also genau zwei zugstabile Gurtvorrichtungen (3, 4) auf, durch die der Druckpunkt festgelegt wird und mittels derer die Orthese in ihrer Kraftwirkung angepasst werden kann. Jede Gurtvorrichtung (3, 4) besteht hierbei vorzugsweise jeweils aus dem Verbindungsgurt (7, 8) der jeweiligen Gurtvorrichtung (3, 4) und aus wenigstens einem Endgurt (9, 10, 11, 12) der jeweiligen Gurtvorrichtung (3, 4).

Das Gurtsystem (2) weist in einer weiteren Ausführungsform eine Kreuzführung (5) für zwei Gurtvorrichtungen (3, 4) auf. Hierbei kreuzen sich die zwei Gurtvorrichtungen (3, 4) im Bereich der Kreuzführung (5) und werden mittels der Kreuzführung (5) zwangsgeführt gehalten, wie beispielsweise in der Fig. 1 dargestellt. Die Gurtvorrichtungen (3, 4) winden sich dabei spiralartig um das mittels der Orthese zu stützende Gelenk und bewirken eine vorteilhafte Einleitung von Zugkräften. Der Druckpunkt liegt somit in dem Bereich, in dem sich die Gurtvorrichtungen (3, 4) kreuzen.

Bei einer Ausführungsform ist alternativ oder zusätzlich zu der Kreuzführung (5) vorgesehen, dass der Verbindungsgurt (7, 8) wenigstens einer Gurtvorrichtung (3, 4) zumindest abschnittsweise zwei Lagen aufweist. Die Lagen sind zumindest an zwei voneinander beabstandeten Verbindungen miteinander verbunden. Dadurch bilden die Lagen zwischen den Verbindungen eine Schlaufe oder Raute aus. In einer bevorzugten Ausführungsform greifen die Schlaufen von zwei Verbindungsgurten (7, 8) ineinander. Durch jede Schlaufe ist somit zumindest eine Lage des Verbindungsgurts (7, 8) einer weiteren Gurtvorrichtung (3, 4) geführt. Dementsprechend können die Gurtvorrichtungen (3, 4) in einer besonders bevorzugten Ausführungsform nur in durch die Länge der Verbindungsgurte (7, 8) gegebenen Grenzen relativ zueinander verschoben werden und werden ansonsten aneinander gehalten.

Der wenigstens eine Endgurt (9, 10, 11, 12) von wenigstens einer Gurtvorrichtung (3, 4) besteht vorzugsweise jeweils aus genau einem proximalen Endgurt (9, 11) und genau einem distalen Endgurt (10, 12) der jeweiligen Gurtvorrichtung (3, 4). Demnach weist die erste Gurtvorrichtung (3) einen proximalen ersten Endgurt (9) und einen distalen ersten Endgurt (10) sowie die zweite Gurtvorrichtung (4) einen proximalen zweiten Endgurt (11) und einen distalen zweiten Endgurt (12) auf. Jede Gurtvorrichtung (3, 4) weist somit zwei Endgurte (9, 10, 11, 12) und einen zwischen den Endgurten angeordneten Verbindungsgurt (7, 8) auf, der die Endgurte (9, 10, 11, 12) miteinander verbindet. Der Verbindungsgurt (7, 8) der jeweiligen Gurtvorrichtung (3, 4) weist dabei einen proximalen Endbereich (13, 15) auf, mittels welchem der jeweilige Verbindungsgurt (7, 8) mit dem proximalen Endgurt (9, 11) der jeweiligen Gurtvorrichtung (3, 4) verbunden ist. Weiter weist der Verbindungsgurt (7, 8) der jeweiligen Gurtvorrichtung einen distalen Endbereich (14, 16) auf, mittels welchem der jeweilige Verbindungsgurt (7, 8) mit dem distalen Endgurt (10, 12) der jeweiligen Gurtvorrichtung (3, 4) verbunden ist. Wie beispielsweise der Fig. 1 zu entnehmen ist, umfasst der erste Verbindungsgurt (7) einen proximalen ersten Endbereich (13), mittels welchem er mit dem proximalen ersten Endgurt (9) winkelverstellbar und lösbar verbunden ist, und einen distalen ersten Endbereich (14), mittels welchem er mit dem distalen ersten Endgurt 10 winkelverstellbar und lösbar verbunden ist. Der zweite Verbindungsgurt (8) weist entsprechend der Anordnung des ersten Verbindungsgurt (7) einen proximalen (15) und einen distalen (16) zweiten Endbereich auf, mittels derer er an einem proximalen zweiten Endgurt (11) bzw. distalen zweiten Endgurt (12) winkelverstellbar und lösbar verbunden ist.

Angaben wie proximal oder distal werden in Bezug auf die Erfindung analog zu Angaben in Bezug auf den Teil des menschlichen Körpers gebraucht, an den die erfindungsgemäße Orthese angelegt werden kann, insbesondere das Kniegelenk.

Bei einer bevorzugten Ausführungsform ist wenigstens ein Endgurt (9, 10, 11, 12) von wenigstens einer Gurtvorrichtung (3, 4) mittels einer Klettverbindung (18, 19, 20, 21) mit dem Verbindungsgurt der jeweiligen Gutvorrichtung (3, 4) winkelverstellbar und lösbar verbunden. Insbesondere sind beide Endgurte der jeweiligen Gurtvorrichtung (3, 4) mittels einer Klettverbindung (18, 19, 20, 21) mit einem Verbindungsgurt (7, 8) dieser Gurtvorrichtung (3, 4) verbunden. Dies gilt vorzugsweise für beide Gurtvorrichtungen (3, 4), so dass an jedem Ende eines jeden Verbindungsgurts (7, 8) jeweils eine Klettverbindung (18, 19, 20, 21) vorhanden ist. Bei zwei Verbindungsgurten (7, 8) sind somit vier Klettverbindungen (18, 19, 20, 21) vorhanden, an denen der Winkel der Gurtvorrichtungen (3, 4) einstellbar ist. Die Winkelverstellung an beiden Enden des Verbindungsgurts (7, 8) ermöglich, dass der der Winkel, in welchem der Verbindungsgurt (7, 8) verläuft, weitgehend vom Winkel, in welchem die Gurtvorrichtung (3, 4) insgesamt verläuft, entkoppelt bleiben kann. Dies ist insbesondere auch von Vorteil in Verbindung mit der Kreuzführung (5).

Die Klettverbindungen (18, 19, 20, 21) zwischen dem Verbindungsgurt (7, 8) und den Endgurten sind vorzugsweise jeweils eine doppelte oder beidseitige Klettverbindung (18, 19, 20, 21). Dabei weist der Verbindungsgurt (7, 8) der jeweiligen Gurtvorrichtung (3, 4) ein Gurtelement (17) mit einer Vorderseite und einer Rückseite auf. An jedem Ende des Gurtelements (17) ist auf der Rückseite jeweils ein Hakenelement für die Klettverbindung (18, 19, 20, 21) und eine korrespondierend zu diesem Hakenelement angeordnete und klappbare Lasche mit einem dem Hakenband zugewandten weiteren Hakenband vorhanden. Die Endgurte (9, 10, 11, 12) weisen dabei beidseitig zumindest abschnittsweise ein Schlaufenband auf, das zwischen den Hakenbändern positionierbar ist. Das Schlaufenband ist vorzugsweise aus Velours. Dabei kann unter dem Endgurt (9, 10, 11, 12) mit Schlaufenband wahlweise ein Endgurt mit einer Schlaufen aufweisenden Oberfläche, insbesondere Veloursoberfläche, oder auch ein Endgurt mit einem auf seiner Oberfläche befestigten Band, welches die Schlaufen aufweist, verstanden werden. Die Endgurte (9, 10, 11, 12) sind mittels der durch die Hakenbänder und die Schlaufenbänder gegebenen Klettverbindung (18, 19, 20, 21) mit dem Verbindungsgurt (7, 8) winkelverstellbar verbindbar. Durch die doppelseitige Klettverbindung (18, 19, 20, 21) ist eine besondere Sicherheit gegen ein unbeabsichtigtes Lösen der Klettverbindung gegeben.

Bei einer Ausführungsform ist alternativ oder zusätzlich zur Klettverbindung (18, 19, 20, 21) wenigstens ein Endgurt von wenigstens einer Gurtvorrichtung (3, 4) mittels einer Schraubverbindung mit dem jeweiligen Verbindungsgurt (7, 8) der jeweiligen Gurtvorrichtung (3, 4) winkelverstellbar verbunden.

Gemäß einer weiteren Ausführungsform ist alternativ oder zusätzlich zur Klettverbindung (18, 19, 20, 21) und/oder zur Schraubverbindung wenigstens ein Endgurt (9, 10, 11, 12) von wenigstens einer Gurtvorrichtung (3, 4) mittels einer Nietverbindung mit dem jeweiligen Verbindungsgurt (7, 8) der jeweiligen Gurtvorrichtung (3, 4) winkelverstellbar verbunden. Die Nietverbindung ermöglicht zwar die Winkelverstellung, hält den Verbindungsgurt (7, 8) und den Endgurt (9, 10, 11, 12) jedoch ansonsten aneinander, sodass keine Längsverstellung des Verbindungsgurts im Bereich der Nietverbindung möglich ist.

Bei der Klettverbindung (18, 19, 20, 21) und bei der Schraubverbindung ist hingegen auch eine Längenverstellung der Gurtvorrichtung (3, 4) im Bereich der jeweiligen Verbindung möglich. Zur Längenanpassung der wenigstens einen Gurtvorrichtung (3, 4) sind bevorzugt unterschiedliche Bereiche wenigstens eines Endgurts (9, 10, 11, 12) der jeweiligen Gurtvorrichtung (3, 4) mit dem Verbindungsgurt (7, 8) der jeweiligen Gurtvorrichtung (3, 4) verbindbar. Alternativ oder zusätzlich ist zur Längenverstellung ein Endgurt (9, 10, 11, 12) der jeweiligen Gurtvorrichtung (3, 4) mittels unterschiedlichen Bereichen des Verbindungsgurts (7, 8) der jeweiligen Gurtvorrichtung (3, 4) verbindbar. Eine weitere Möglichkeit, die Länge der Gurtvorrichtung (3, 4) anzupassen, besteht darin, einen Gurtabschnitt des Endgurts (9, 10, 11, 12) umzuschlagen. Der Umschlag führt zur Bildung einer Schlaufe, die je nach Länge des umgeschlagenen Gurtabschnitts unterschiedlich lang ist. Gemäß einer bevorzugten Ausführungsform weist entsprechend wenigstens ein Endgurt (9, 10, 11, 12) wenigstens einer Gurtvorrichtung (3, 4) zur Längenverstellung einen unteren Gurtabschnitt und einen auf den unteren Gurtabschnitt umgeschlagenen oberen Gurtabschnitt auf, wobei mit diesen Gurtabschnitten eine längenverstellbare Schlaufe ausgebildet ist.

Das freie Ende des oberen Gurtabschnitts muss zur Bildung der Schlaufe am unteren Gurtabschnitt befestigt sein. Vorzugsweise ist das freie Ende des oberen Gurtabschnitts lösbar am unteren Gurtabschnitt befestigt, um die Orthese (1) mit entspannter Gurtvorrichtung (3, 4) anlegen und nachfolgend die Gurtvorrichtung (3, 4) unkompliziert in voreingestellter Länge spannen zu können. Hierfür weist die Gurtvorrichtung (3, 4) am oberen Gurtabschnitt des Endgurts in einer bevorzugten Ausführungsform einen Schnellverschluss (40) auf, mittels dem die Schlaufe lösbar geschlossen ist. Der Schnellverschluss (40) weist vorzugsweise einen Magneten auf, der den Schnellverschluss (40) magnetisch in eine Schließposition zieht und in der Schließposition verriegelt. Dadurch wirkt der Schnellverschluss (40) einem unbeabsichtigten Öffnen des Schnellverschlusses entgegen. Für die Längeneinstellung der Schlaufe ist bevorzugt vorgesehen, dass ein dem oberen Gurtabschnitt angeordnetes Ende des Endgurts (9, 10, 11, 12) mittels einer Klettverbindung (18, 19, 20, 21) in einem zu Längsverstellung des Schlaufe wählbaren Bereich am unteren Ende des Endgurts (9, 10, 11, 12) befestigt ist.

In einer Ausführungsform sind die Enden aller oberen Gurtabschnitte jeweils mittels einer Klettverbindung an einem zugeordneten unteren Gurtabschnitt festgelegt, so etwa das Ende eines oberen Gurtabschnitts (38) mittels einer Klettverbindung (39) an einem unteren Gurtabschnitt (37). Die Schlaufe ist somit geschlossen, wobei ein Schnellverschluss (40) eine zusätzliche Möglichkeit der Öffnung oder des Verschlusses der Schlaufe bietet, ohne jedoch die Klettverbindung (39) lösen. Der Schnellverschluss (40) weist hierfür miteinander verrastbare Verschlusselemente auf, die mittels eines Magneten gesichert aneinander gehalten sind. Zusätzlich kann eine Zugschlaufe (41) vorgesehen sein, die beim Zusammenziehen vor dem Verrasten hilft.

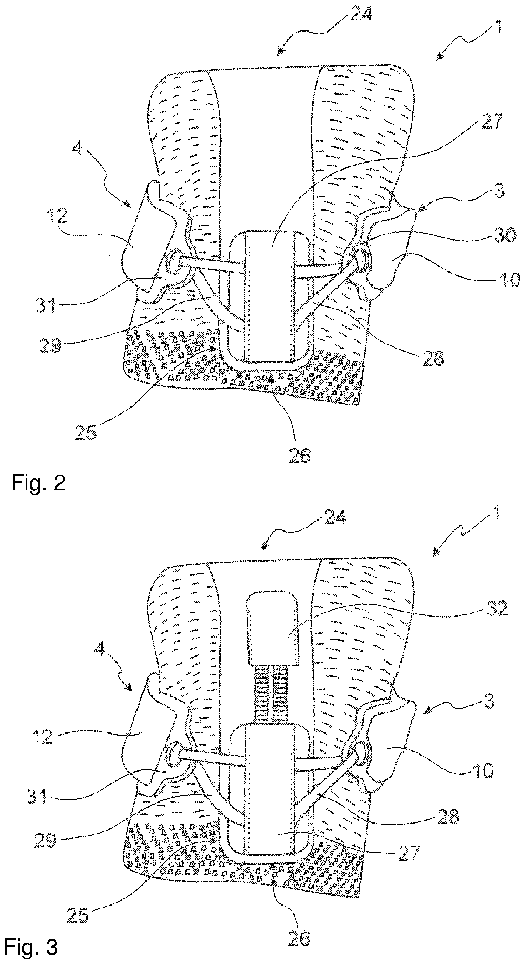

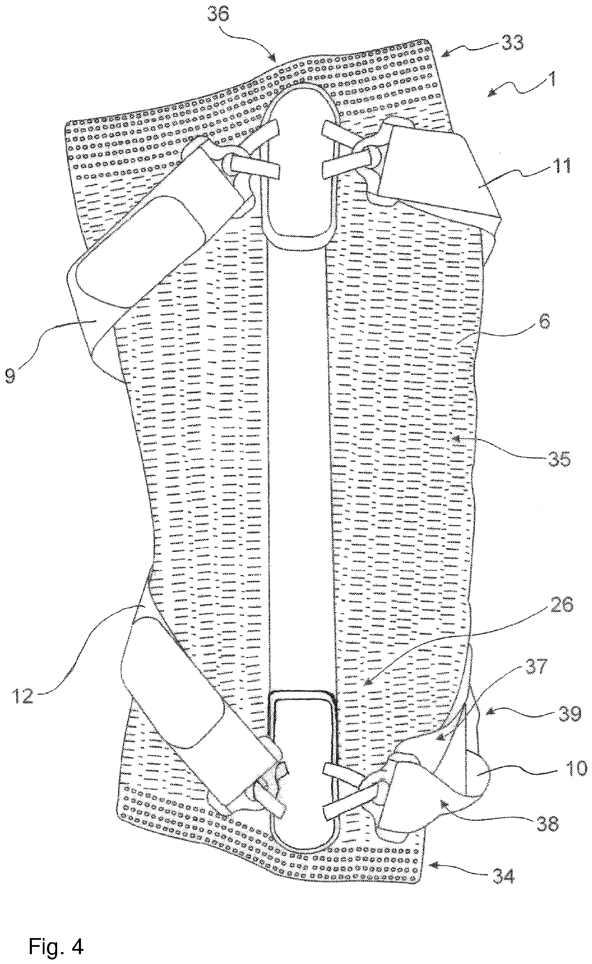

In der Erfindung ist vorgesehen, dass die Orthese (1) eine Manschette (6) aufweist, an der das Gurtsystem befestigt ist, wie beispielsweise der Fig. 1 und Fig. 2 zu entnehmen ist. Das Gurtsystem besteht dabei aus genau einer ersten Gurtvorrichtung (3) mit einem ersten Verbindungsgurt (7) und genau einer zweiten Gurtvorrichtung (4) mit einem zweiten Verbindungsgurt (8). Dabei ist weiter vorgesehen, dass beide Gurtvorrichtungen (3, 4) einander im Bereich der Kreuzführung (5) kreuzen und in diesem Bereich mittels an der Manschette (6) gehalten werden. Ferner wird je eine Gurtvorrichtungen (3, 4) jeweils an einem proximalen Bereich und an einem distalen Bereich der Manschette (6) befestigt. Des Weiteren ist in der Erfindung vorgesehen, dass das Gurtsystem Befestigungsvorrichtungen mit Seilen (28, 29) aufweist, mittels denen beide Gurtvorrichtungen (3, 4) an der Manschette (6) befestigt sind. Die Seile (28, 29) sind insbesondere Bänder oder bandhafte Verbindungen. Ferner weist gemäß der Erfindung das Gurtsystem Umlenkösen auf, an welchen die Endgurte der Gurtvorrichtungen gehalten sind, wobei jedes Seil (28, 29) jeder Befestigungsvorrichtung (26) jeweils durch eine Umlenköse (30, 31) hindurchragt und diese Umlenköse (30, 31) rotationsbeweglich an der jeweiligen Befestigungsvorrichtung (26, 36) hält. Wie beispielsweise in Fig. 4 dargestellt, weist jeder Endgurt (9, 10, 11, 12) jeweils einen unteren Gurtabschnitt und einen oberen Gurtabschnitt auf, wobei mittels den Gurtabschnitten jeweils eine Schlaufe ausgebildet ist, welche durch die jeweilige Umlenköse (30, 31) geführt ist.

Die Manschette (6) ist im Wesentlichen aus einem flexiblen textilen Material gefertigt vorzugsweise ist diese aus einem Gewebe, einem Gestrick oder einem Gewirke gebildet. In einer besonders bevorzugten Ausführungsform handelt es sich um ein elastisches Material. Die Orthese (1) ist daher sehr leicht und komfortabel zu tragen. Medial und lateral sind, wie in Fig. 1 dargestellt, in einer Ausführungsform zusätzlich sich in proximal-distaler Richtung erstreckende Stabilisierungselemente (23, 24) in Form von Schienen in die Manschette (6) eingearbeitet. Diese geben eine Stütze in proximal-distaler Richtung, jedoch lassen sie eine Beugung etwa des Knies in vertikal-dorsaler Richtung durch Biegsamkeit oder durch wenigstens ein Gelenk zu. Die Stabilisierungselemente (23, 24) nehmen die Vertikalkomponenten der eingeleiteten Gurtkräfte auf, wodurch die Bandage bzw. Orthese (1) nicht gestaucht wird. Die Stabilisierungselemente können eine flexible Kunststoffschiene, eine Flachspiralfeder und/oder eine Metallschiene sein. In einer bevorzugten Ausführungsform ist in proximal-distaler Richtung ein erstes Stabilisierungselement (23) in die Manschette (6) eingearbeitet, wie in Fig. 1 dargestellt. Das erste Stabilisierungselement (23) ist insbesondere eine flexible Kunststoffschiene oder eine Flachspiralfeder. Die Kreuzführung (5) ist in etwa über einem mittleren Bereich des ersten Stabilisierungselementes (23) angeordnet. An einer dem ersten Stabilisierungselement (23) gegenüberliegenden Seite der Orthese (1), auf welcher ein zweites Stabilisierungselement (24), insbesondere in Form einer Metallschiene mit wenigstens einem Gelenk, in die Manschette eingearbeitet ist, wie beispielsweise der Fig. 2 zu entnehmen ist. Das zweite Stabilisierungselement (24) verläuft vorzugsweise in proximal-distaler Richtung und ist besonders bevorzugt von einem Schlaufenband verdeckt, auf welchem mittels einer Klettverbindung (25) eine distale Befestigungsvorrichtung (26) befestigt ist. Die eine Befestigungsvorrichtung (26, 36) kann vertikal versetzbar an der Manschette (6) befestigt sein. Dadurch kann die Anordnung der Gurtvorrichtungen (3, 4) weiter optimiert werden. Insbesondere weist die Orthese genau zwei Befestigungsvorrichtungen (26, 36) auf, die beide vertikal versetzbar an der Manschette (6) befestigt sind.

Die wenigstens eine Befestigungsvorrichtung (26, 36) weist in einer Ausführungsform ein Basisteil (27) auf, an welchem jedes Seil (28, 29) der jeweiligen Befestigungsvorrichtung (26, 36) an jeweils zwei vertikal voneinander beabstandeten Stellen gehalten ist und zusammen mit dem Basisteil (27) eine Schlaufe bildet, wie beispielsweise der Fig. 2 zu entnehmen ist. Vorzugsweise weisen beide Befestigungsvorrichtungen (26, 36) jeweils ein Basisteil (27) auf. Bei einer besonders bevorzugten Ausführungsform weist das Basisteil (27) ein Hakenband und die Manschette (6) zumindest abschnittsweise ein Schlaufenband auf, siehe beispielsweise Fig. 2. Das Schlaufenband ist hierbei vorzugsweise eine Veloursoberfläche an der Manschette (6). Das Hakenband und das Schlaufenband bilden dabei zusammen eine Klettverbindung, mittels der das Basisteil (27) an der Manschette (6) befestigbar und positionierbar, insbesondere befestigt und positioniert, ist. Die Befestigungsvorrichtungen (26, 36) können daher bei Bedarf von der Manschette (6) abgenommen werden.

Besonders bevorzugt sind die Stellen, an denen ein Seil (28, 29) einer Befestigungsvorrichtung (26, 36) gehalten ist, jeweils paarweise mit den Stellen, an denen das andere Seil (28, 29) dieser Befestigungsvorrichtung gehalten ist, am Basisteil (27) angeordnet. Paarweise angeordnete Stellen weisen dabei jeweils eine gleiche proximale oder distale Position an der Manschette (6) auf. Dadurch ist ein Kräftegleichgewicht gegeben.

Bei einer vorteilhaften Ausführungsform ist vorgesehen, dass jedes Seil (28, 29) wenigstens einer Befestigungsvorrichtung (26, 36) ausgehend von jeweils einer der vertikal voneinander beabstandeten Stellen durch das Basisteil hindurch geführt ist und zum Spannen der jeweiligen Gurtvorrichtung (3, 4) mit einem Ende des Seils aus dem Basisteil (27) herausgeführt ist, wie beispielsweise Fig. 3 zu entnehmen ist. Dabei ist vorzugsweise das jeweilige Seil (28, 29) in dem Basisteil (27) um einen Winkel zwischen 80° und 100°, insbesondere zwischen 85° und 95°, umgelenkt. Weiter ist bevorzugt vorgesehen, dass die jeweilige Befestigungsvorrichtung (26, 36) ein dem Basisteil (27) zugeordnetes Greifteil (32) aufweist, welches mit den herausgeführten Enden der Seile der jeweiligen Befestigungsvorrichtung (26, 36) verbunden ist. Enden der Seile verlaufen somit in etwa parallel vom Basisteil (27) zum Greifteil (32). Beide Seile können daher gemeinsam mittels des Greifteils (32) gestrafft oder gelockert werden, um bei Bedarf die Länge der mit den Seilen (28, 29) gebildeten Schlaufen zu verändern. Zur Fixierung dieser Länge ist vorgesehen, dass das Greifteil (32) eine Greiflasche oder ein Ring ist und lösbar an der Manschette (6) fixiert ist, insbesondere mittels einer Klettverbindung. Hierfür weist wiederum vorzugsweise die Greiflasche ein Hakenband und die Manschette (6) ein Schlaufenband auf, wie beispielsweise der Fig. 3 zu entnehmen ist.

In einer Ausführungsform ist innerhalb der Orthese (1) ein Ring angeordnet. Dieser Ring bildet einen Anlagebereich (35) für die Kniescheibe, wodurch die Position der Orthese (1) zur Kniescheibe einerseits besser positioniert werden kann und der Tragekomfort der Orthese (1) verbessert wird. Der Anlagebereich (35) befindet sich annährend mittig der Manschette (6), wie beispielsweise der Fig. 4 zu entnehmen ist. Der Ring bzw. der Anlagebereich (35) befindet sich in Bezug auf die Orthese (1) innenliegend, sodass der Ring verdeckt angeordnet an der Orthese (1) ist.

Diese und andere Ausführungsformen der vorliegenden Erfindung werden in der Beschreibung, Figuren und den Beispielen offenbart und sind durch diese umfasst. Die in der vorstehenden Beschreibung und in den Ansprüchen genannten Merkmale sind weiterhin in einer beliebigen Auswahl kombinierbar und die im Rahmen der Erfindung sinnvollen Merkmalskombinationen als offenbart zu betrachten. Weitere Literatur über eine bekannte der Materialien, Verfahren und Anwendungen die in Übereinstimmung mit der vorliegenden Erfindung verwendet werden können, können aus öffentlichen Bibliotheken und Datenbanken, beispielsweise unter Verwendung elektronischer Geräte aufgerufen werden. Ein vollständigeres Verständnis der Erfindung kann durch Bezugnahme auf die Figuren und Beispiele erhalten werden, die zum Zweck der Illustration bereitgestellt wurden und den Umfang der Erfindung nicht beschränken sollen.

### Beispiele

### Beispiel 1: Aufbau einer beispielhaften Gonarthrose-Orthese

Eine beispielhafte Ausführungsform der erfindungsgemäßen Orthese (1) ist in den Fig. 1, Fig. 2 und den Fig. 4 und 5 dargestellt.

In Fig. 1 weist die Orthese (1) ein Gurtsystem (2) mit einer ersten Gurtvorrichtung (3), einer zweiten Gurtvorrichtung (4), einer Kreuzführung (5) für die Gurtvorrichtungen (3, 4) und einer Manschette (6) auf. Die Gurtvorrichtungen (3, 4) kreuzen sich im Bereich der Kreuzführung (5) und sind in diesem Bereich mittels der Kreuzführung (5) an der Manschette (6) gehalten. Dabei umfasst
(a) die erste Gurtvorrichtung (3)
   i) einen ersten Verbindungsgurt (7);
   ii) einen proximalen ersten Endgurt (9); und
   iii) einen distalen ersten Endgurt 10; und
b) die zweite Gurtvorrichtung (4)
   i) einen zweiten Verbindungsgurt (8), welcher den ersten Verbindungsgurt (7) im Bereich der Kreuzführung (5) kreuzt;
   ii) einen proximalen zweiten Endgurt (11); und
   iii) einen distalen zweiten Endgurt (12).

Der erste Verbindungsgurt (7) weist einen proximalen ersten Endbereich (13) auf, mittels welchem er mit dem proximalen ersten Endgurt (9) winkelverstellbar und lösbar verbunden ist. Weiterhin weist der erste Verbindungsgurt (7) einen distalen ersten Endbereich (14) auf, mittels welchem er mit dem distalen ersten Endgurt (10) winkelverstellbar und lösbar verbunden ist.

Der zweite Verbindungsgurt (8) weist entsprechend einen proximalen zweiten Endbereich (15) und einen distalen zweiten Endbereich (16) auf. Der proximale zweite Endbereich (15) verbindet den Verbindungsgurt (8) mit dem proximalen zweiten Endgurt (11), wobei die Verbindung winkelverstellbar und lösbar ist. Der distale zweite Endbereich (16) ermöglicht eine entsprechende Verbindung des Verbindungsgurtes (8) mit dem distalen zweiten Endgurt (12), welche auch winkelverstellbar und lösbar verbunden ist.

Dabei besteht der erste Verbindungsgurt (7) aus einem ersten Gurtelement (17), aus jeweils einer klappbare Lasche an jedem Ende des Gurtelements (17), also im proximalen ersten Endbereich (13) und im distalen ersten Endbereich (14), und aus Hakenbändern in den Endbereichen (13, 14) sowohl am Gurtelement (17) als auch an der klappbaren Lasche. Die Hakenbänder am jeweiligen Endbereich (13 oder 14) sind dabei einander zugewandt und halten beidseitig Schlaufenbänder der ersten Endgurte (9, 10), welche jeweils in Form einer beidseitigen Veloursoberfläche der Endgurte (9, 10) gegeben sind. Durch die Hakenbänder und die Schlaufenbänder sind eine proximale erste Klettverbindung (18) und eine distale erste Klettverbindung (19) zwischen den ersten Endgurten (9, 10) und dem ersten Verbindungsgurt (7) gegeben. Entsprechend weist der zweite Verbindungsgurt (8) ein zweites Gurtelement (20) auf und sind eine proximale zweite Klettverbindung (21) und eine distale zweite Klettverbindung (22) zwischen dem zweiten Verbindungsgurt (8) und den zweiten Endgurten (11, 12) gegeben.

Die Manschette (6) weist in proximal-distaler Richtung ein in diese eingearbeitetes erstes Stabilisierungselement (23) auf, wobei dieses vorzugsweise eine flexible Kunststoffschiene oder eine Flachspiralfeder ist. Die Kreuzführung (5) ist in etwa über einem mittleren Bereich des ersten Stabilisierungselementes (23) angeordnet.

In Fig. 2 zeigt eine weitere Darstellung der erfindungsgemäßen Orthese (1), nämlich die dem ersten Stabilisierungselement (23) gegenüberliegenden Seite. Auf dieser Seite ist ein zweites Stabilisierungselement (24) in die Manschette (5) eingearbeitet. Dieses zweite Stabilisierungselement (24) besteht vorzugsweise aus einer Metallschiene mit wenigstens einem Gelenk. Das Stabilisierungselement (24) verläuft in proximal-distaler Richtung und ist vorzugsweise von einem Schlaufenband verdeckt, auf welchem mittels einer Klettverbindung (25) eine distale Befestigungsvorrichtung (26) befestigt ist.

An der distalen Befestigungsvorrichtung (26) ist ein Basisteil (27) angeordnet, welches rückseitig mit einem Hakenband versehen ist, wobei mittels dieses Hakenbandes und des zuvor genannten Schlaufenbandes die Klettverbindung (25) gebildet wird. Mittels dieser Klettverbindung (25) kann das Basisteil (27) in proximal-distaler Richtung an unterschiedlichen Stellen an der Manschette (6) befestigt werden.

Die distale Befestigungsvorrichtung (26) weist weiter Seile (28, 29) oder alternativ ein gemeinsames Seil auf. Die Seile (28, 29) sind an beiden Enden beabstandet voneinander am Basisteil (27) befestigt. Dabei sind die Seile (28, 29) korrespondierend zueinander angeordnet, sodass ein Kräftegleichgewicht hergestellt ist. Das Seil (28) ist durch eine distale erste Umlenköse (30) geführt, an welcher die erste Gurtvorrichtung (3) mittels des distalen ersten Endgurts (10) gehalten ist. Das Seil (29) ist durch eine distale zweite Umlenköse (31) geführt, an welcher die zweite Gurtvorrichtung (4) mittels des distalen zweiten Endgurts (12) gehalten ist.

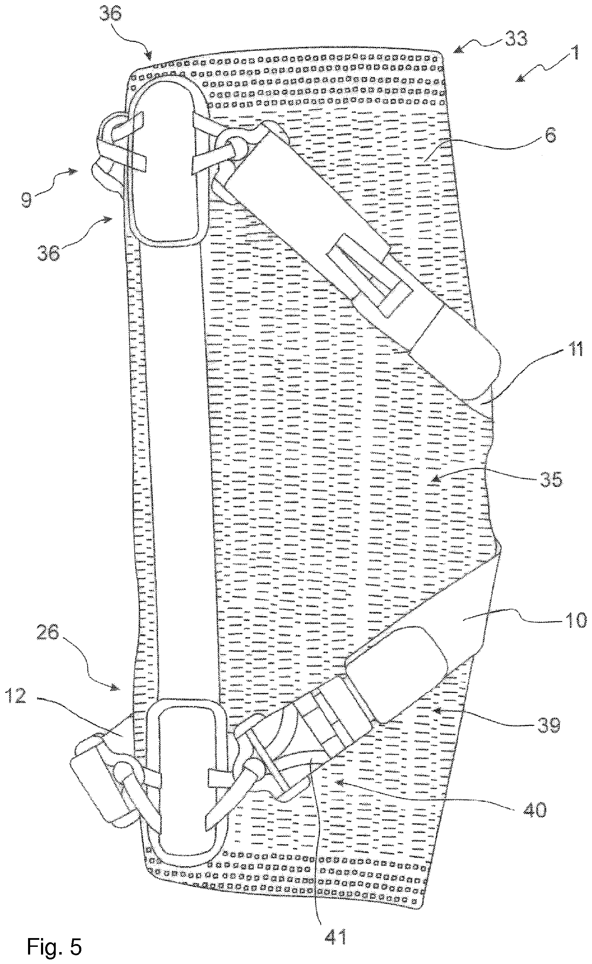

Zusätzlich zu der distalen Befestigungsvorrichtung (26) im distalen Bereich (34) der Manschette (6) ist eine entsprechend ausgebildete proximale Befestigungsvorrichtung (36) im proximalen Bereich (33) der Manschette (6) vorgesehen. Dies ist beispielsweise den Fig. 4 und Fig. 5 zu entnehmen, die die Orthese (1) in zwei Ansichten in ihrer vollständigen proximal-distalen Erstreckung darstellen, nämlich von einem proximalen Bereich (33) der Manschette (6) bis zu einem distalen Bereich (34) der Manschette (6). Die beiden Gurtvorrichtungen (3, 4) sind somit an beiden Befestigungsvorrichtungen (26, 36) gehalten.

Jeder Endgurt (9, 10, 11 und 12) weist des Weitern jeweils einen unteren Gurtabschnitt und einen oberen Gurtabschnitt auf, wobei mittels den Gurtabschnitten jeweils eine Schlaufe ausgebildet ist, welche durch die jeweilige Umlenköse (30, 31) geführt ist. Aus Gründen der Übersichtlichkeit ist in der Fig. 4 vom distalen ersten Endgurt (10) lediglich der untere Gurtabschnitt mit dem Bezugszeichen (37) und der oberen Gurtabschnitt mit dem Bezugszeichen (38) bezeichnet. Die Enden aller oberen Gurtabschnitte sind jeweils mittels einer Klettverbindung am zugeordneten unteren Gurtabschnitt festgelegt, so etwa das Ende des oberen Gurtabschnitts (38) mittels einer Klettverbindung (39) am unteren Gurtabschnitt (37). Die Schlaufe ist somit geschlossen, wobei mittels eines Schnellverschlusses (40) eine zusätzliche Möglichkeit gegeben ist, die Schlaufe zu öffnen und zu schließen, ohne die Klettverbindung (39) lösen zu müssen. Der Schnellverschluss (40) weist hierfür miteinander verrastbare Verschlusselemente auf, die mittels eines Magneten gesichert aneinander gehalten sind. Zusätzlich ist eine Zugschlaufe (41) vorgesehen, die beim Zusammenziehen vor dem Verrasten hilft.

Weiterhin ist wie in den Fig. 4 und 5 dargestellt etwa mittig der Manschette (6) ein Anlagebereich (35) für die Kniescheibe in Form eines verdeckt angeordneten Rings ausgebildet.

### Beispiel 2: Orthese mit alternativer Seilbefestigung

Bei der alternativen Ausführungsform die beispielhaft in Fig. 3 dargestellt ist, sind die Seile (28, 29) an der proximalen Seite durch das Basisteil (27) hindurchgeführt bis zu einem distalen Greifteil (32) und nicht am Basisteil (27) befestigt. Das Griffteil (32) ermöglicht die Längen von Schlaufen, welche durch die Seile (28, 29) und das Basisteil (27) gebildet werden, zu verändern. Das distale Greifteil (32) kann dabei mittels einer lösbaren Klettverbindung auf einer Veloursoberfläche an der Manschette (6) festgelegt werden.

### Beispiel 3: Orthese mit fest angebrachter Seilbefestigung

In einer Ausführungsform der vorliegenden erfindungsmäßen Orthese weißt die Orthese fest angeordnete Seilbefestigungen auf. Hierfür sind die Basisteile (27) fest mit der Tasche des Stabilisierungselementes (24) am proximalen und distalen Ende verbunden.

## Patentansprüche

1. Orthese, insbesondere Gonarthroseorthese zur Entlastung des medialen oder lateralen Kniekompartiments des menschlichen Knies, mit einem Gurtsystem (2), wobei das Gurtsystem (2) wenigstens eine zugstabile Gurtvorrichtung (3, 4) aufweist, wobei jede Gurtvorrichtung (3,4) jeweils wenigstens einen Endgurt (9, 10, 11, 12) aufweist, wobei jede Gurtvorrichtung (3, 4) jeweils mindestens einen Verbindungsgurt (7, 8) aufweist, der winkelverstellbar mit jedem Endgurt (9, 10, 11, 12) der jeweiligen Gurtvorrichtung (3, 4) verbunden ist, wobei die Orthese (1) eine Manschette (6) aufweist, an der das Gurtsystem (2) befestigt ist, dass die wenigstens eine Gurtvorrichtung (3, 4) aus genau einer ersten Gurtvorrichtung (3) mit einem ersten Verbindungsgurt (7) und genau einer zweiten Gurtvorrichtung (4) mit einem zweiten Verbindungsgurt (8) besteht, dass beide Gurtvorrichtungen (3, 4) einander kreuzen und jeweils an einem proximalen Bereich (33) und an einem distalen Bereich (34) der Manschette (6) befestigt sind, **dadurch gekennzeichnet, dass** das Gurtsystem (2) Befestigungsvorrichtungen (26, 36) mit Seilen (28, 29) aufweist, mittels denen beide Gurtvorrichtungen (3, 4) an der Manschette (6) befestigt sind, und dass das Gurtsystem (2) Umlenkösen (30, 31) aufweist, an welchen die Endgurte (9, 10, 11, 12) der Gurtvorrichtungen (3, 4) gehalten sind, wobei jedes Seil (28, 29) jeder Befestigungsvorrichtung (26, 36), jeweils durch eine Umlenköse (30, 31) hindurchragt und diese Umlenköse (30, 31) in einem Freiheitsgrad der Translation und zwei Freiheitsgraden der Rotation an der jeweiligen Befestigungsvorrichtung (26, 36) hält.

2. Orthese nach Anspruch 1, **dadurch gekennzeichnet, dass** das Gurtsystem (2) eine Kreuzführung (5) für zwei Gurtvorrichtungen (3,4) aufweist, wobei die zwei Gurtvorrichtungen (3, 4) sich im Bereich der Kreuzführung (5) kreuzen und mittels der Kreuzführung (5) zwangsgeführt gehalten sind.

3. Orthese nach Anspruch 1 oder 2, **dadurch gekennzeichnet, dass** der wenigstens eine Endgurt (9, 10, 11, 12) von wenigstens einer Gurtvorrichtung (3, 4) jeweils aus genau einem proximalen Endgurt (9, 11) der jeweiligen Gurtvorrichtung (3, 4) und genau einem distalen Endgurt (10, 12) der jeweiligen Gurtvorrichtung (3, 4) besteht und dass der Verbindungsgurt (7, 8) der jeweiligen Gurtvorrichtung (3,4) einen proximalen Endbereich (13, 15) aufweist, mittels welchem der jeweilige Verbindungsgurt (7, 8) mit dem proximalen Endgurt (9, 11) der jeweiligen Gurtvorrichtung (3, 4) verbunden ist, und einen distalen Endbereich (14, 16) aufweist, mittels welchem der jeweilige Verbindungsgurt (7, 8) mit dem distalen Endgurt (10, 12) der jeweiligen Gurtvorrichtung (3, 4) verbunden ist.

4. Orthese nach einem der Ansprüche 1 bis 3, **dadurch gekennzeichnet, dass** wenigstens ein Endgurt (9, 10, 11, 12) von wenigstens einer Gurtvorrichtung (3, 4) mittels einer Klettverbindung (18, 19, 21, 22) mit dem Verbindungsgurt der jeweiligen Gurtvorrichtung (3, 4) winkelverstellbar und lösbar verbunden ist.

5. Orthese nach Anspruch 4, **dadurch gekennzeichnet, dass** der Verbindungsgurt (7, 8) der jeweiligen Gurtvorrichtung (3,4) ein Gurtelement mit einer Vorderseite und einer Rückseite aufweist und an jedem Ende des Gurtelements (17, 20) auf der Rückseite jeweils ein Hakenband für die Klettverbindung (18, 19, 21, 22) und eine korrespondierend zu diesem Hakenband angeordnete und klappbare Lasche mit einem dem Hakenband zugewandten weiteren Hakenband aufweist, dass die Endgurte (9, 10, 11, 12) beidseitig zumindest abschnittsweise ein Schlaufenband aufweisen, das zwischen den Hakenbändern positionierbar ist, und dass die Endgurte (9, 10, 11, 12) mittels der durch die Hakenbänder und die Schlaufenbänder gegebenen Klettverbindung (18, 19, 21, 22) mit dem Verbindungsgurt (7, 8) winkelverstellbar verbindbar sind.

6. Orthese nach einem der Ansprüche 1 bis 5, **dadurch gekennzeichnet, dass** wenigstens ein Endgurt (9, 10, 11, 12) von wenigstens einer Gurtvorrichtung (3, 4) mittels einer Schraubverbindung oder mittels einer Nietverbindung mit dem jeweiligen Verbindungsgurt (7, 8) der jeweiligen Gurtvorrichtung (3, 4) winkelverstellbar verbunden ist.

7. Orthese nach einem der Ansprüche 1 bis 6, **dadurch gekennzeichnet, dass** wenigstens ein Endgurt (9, 10, 11, 12) wenigstens einer Gurtvorrichtung (3, 4) zur Längenverstellung einen unteren Gurtabschnitt (37) und einen auf den unteren Gurtabschnitt umgeschlagenen oberen Gurtabschnitt (38) aufweist, wobei mit diesen Gurtabschnitten (37, 38) eine längenverstellbare Schlaufe ausgebildet ist.

8. Orthese nach Anspruch 7, **dadurch gekennzeichnet, dass** die Gurtvorrichtung (3, 4) am oberen Gurtabschnitt (38) des Endgurts (9, 10, 11, 12) einen Schnellverschluss (40) aufweist, mittels dem die Schlaufe lösbar geschlossen ist, vorzugsweise wobei der Schnellverschluss (40) einen Magneten aufweist, der den Schnellverschluss (40) magnetisch in eine Schließposition zieht und in der Schließposition verriegelt.

9. Orthese nach Anspruch 7 oder 8, **dadurch gekennzeichnet, dass** ein am oberen Gurtabschnitt (38) angeordnetes Ende des Endgurts (9, 10, 11, 12) mittels einer Klettverbindung (39) in einem zur Längenverstellung der Schlaufe wählbaren Bereich befestigt ist.

10. Orthese nach einem der Ansprüche 1 bis 9, **dadurch gekennzeichnet, dass** wenigstens eine Befestigungsvorrichtung (26, 36) vertikal versetzbar an der Manschette (6) befestigt ist.

11. Orthese nach einem der Ansprüche 1 bis 10, **dadurch gekennzeichnet, dass** wenigstens eine Befestigungsvorrichtung (26, 36) ein Basisteil (27) aufweist, an welchem jedes Seil (28, 29) der jeweiligen Befestigungsvorrichtung (26, 36) an jeweils zwei vertikal voneinander beabstandeten Stellen gehalten ist und zusammen mit dem Basisteil (27) jeweils eine Schlaufe bildet.

12. Orthese nach Anspruch 11, **dadurch gekennzeichnet, dass** das Basisteil (27) ein Hakenband aufweist, dass die Manschette (6) zumindest abschnittsweise ein Schlaufenband aufweist und dass das Hakenband und das Schlaufenband zusammen eine Klettverbindung (25) bilden, mittels der das Basisteil (27) an der Manschette (6) befestigbar und positionierbar ist.

13. Orthese nach Anspruch 11 oder 12 **dadurch gekennzeichnet, dass** die Stellen, an denen ein Seil (2, 29) einer Befestigungsvorrichtung (26, 36) gehalten ist, jeweils paarweise mit den Stellen an denen das andere Seil (28, 29) dieser Befestigungsvorrichtung (26, 36) gehalten ist, am Basisteil (27) angeordnet sind.

14. Orthese nach einem der Ansprüche 12 bis 13, **dadurch gekennzeichnet, dass** jedes Seil (28, 29) wenigstens einer Befestigungsvorrichtung (26, 36) ausgehend von jeweils einer der vertikal voneinander beabstandeten Stellen durch das Basisteil (27) hindurch geführt ist, dabei in dem Basisteil (27) um einen Winkel zwischen 80° und 100° umgelenkt ist und zum Spannen der jeweiligen Gurtvorrichtung (3, 4) mit einem Ende des Seils (28, 29) aus dem Basisteil (27) herausgeführt ist und dass die jeweilige Befestigungsvorrichtung (26, 36) ein dem Basisteil (27) zugeordnetes Greifteil (32) aufweist, welches mit den herausgeführten Enden der Seile (28, 29) der jeweiligen Befestigungsvorrichtung (26, 36) verbunden ist.

## Claims

1. Orthosis, in particular gonarthrosis orthosis for relieving the medial or lateral knee compartment of the human knee, with a belt system (2), wherein the belt system (2) includes at least one tension-stable belt device (3, 4), wherein each belt device (3, 4) comprise at least one end belt (9, 10, 11, 12) each, wherein each belt device (3, 4) comprise at least one connecting belt (7, 8) each, which is connected to each end belt (9, 10, 11, 12) of the respective belt device (3, 4) in an angularly adjustable manner, wherein
the orthosis (1) comprise a cuff (6) to which the belt system (2) is attached, that the at least one belt device (3, 4) consists of exactly one first belt device (3) with a first connecting belt (7) and exactly one second belt device (4) with a second connecting belt (8), that both belt devices (3, 4) cross each other and are each attached to a proximal region (33) and to a distal region (34) of the cuff (6), **characterized in**
**that** the belt system (2) includes fastening devices (26, 36) with ropes (28, 29) by means of which both belt devices (3, 4) are attached to the cuff (6), and that the belt system (2) comprise deflection eyelets (30, 31) at which the end belts (9, 10, 11, 12) of the belt devices (3, 4) are held, wherein each rope (28, 29) of each fastening device (26, 36) projects through a deflection eyelet (30, 31) and holds this deflection eyelet (30, 31) in one degree of freedom of translation and two degrees of freedom of rotation on the respective fastening device (26, 36).

2. Orthosis according to claim 1, **characterized in that** the belt system (2) comprise a cross guide (5) for two belt devices (3, 4), wherein the two belt devices (3, 4) cross each other in the region of the cross guide (5) and are held in a forcibly guided manner by means of the cross guide (5).

3. Orthosis according to claim 1 or 2, **characterized in that** the at least one end belt (9, 10, 11, 12) of at least one belt device (3, 4) each comprises of exactly one proximal end belt (9, 11) of the respective belt device (3, 4) and exactly one distal end belt (10, 12) of the respective belt device (3, 4), and that the connecting belt (7, 8) of the respective belt device (3, 4) has a proximal end region (13, 15) by means of which the respective connecting belt (7, 8) is connected to the proximal end belt (9, 11) of the respective belt device (3, 4), and has a distal end region (14, 16) by means of which the respective connecting belt (7, 8) is connected to the distal end belt (10, 12) of the respective belt device (3, 4).

4. Orthosis according to any of claims 1 to 3, **characterized in that** at least one end belt (9, 10, 11, 12) of at least one belt device (3, 4) is angularly adjustably and releasably connected to the connecting belt of the respective belt device (3, 4) by means of a hook-and-loop connection (18, 19, 21, 22).

5. Orthosis according to claim 4, **characterized in that** the connecting belt (7, 8) of the respective belt device (3, 4) comprises a belt element with a front side and a rear side and at each end of the belt element (17, 20) on the rear side each includes a hook band for the hook-and-loop connection (18, 19, 21, 22) and a foldable tab arranged corresponding to this hook band, which comprises a further hook band facing said hook band, that the end belts (9, 10, 11, 12) on both sides at least sectionally include a loop band which can be positioned between the hook bands, and that the end belts (9, 10, 11, 12) can be connected to the connecting belt (7, 8) in an angularly adjustable manner by means of the hook-and-loop connection (18, 19, 21, 22) given by the hook bands and the loop bands.

6. Orthosis according to any of claims 1 to 5, **characterized in that** at least one end belt (9, 10, 11, 12) of at least one belt device (3, 4) in an angularly adjustable manner is connected to the respective connecting belt (7, 8) of the respective belt device (3, 4) by means of a screw connection or by means of a rivet connection.

7. Orthosis according to any of claims 1 to 6, **characterized in that** for length adjustment at least one end belt (9, 10, 11, 12) of at least one belt device (3, 4) comprises a lower belt portion (37) and an upper belt portion (38) folded over onto the lower belt portion, wherein a length-adjustable loop is formed with these belt portions (37, 38).

8. Orthosis according to claim 7, **characterized in that** at the upper belt portion (38) of the end belt (9, 10, 11, 12) the belt device (3, 4) comprises a quick-release fastener (40) by means of which the loop is releasably closed, preferably wherein the quick-release fastener (40) includes a magnet which magnetically pulls the quick-release fastener (40) into a closed position and locks it in the closed position.

9. Orthosis according to claim 7 or 8, **characterized in that** one end of the end belt (9, 10, 11, 12) arranged on the upper belt portion (38) is fastened by means of a hook-and-loop connection (39) in a region selectable for the length adjustment of the loop.

10. Orthosis according to any of claims 1 to 9, **characterized in that** the at least one fastening device (26, 36) is attached to the cuff (6) in a vertically displaceable manner.

11. Orthosis according to any of claims 1 to 10, **characterized in that** at least one fastening device (26, 36) comprises a base part (27) on which each rope (28, 29) of the respective fastening device (26, 36) is held at two vertically spaced points each and together with the base part (27) each forms a loop.

12. Orthosis according to claim 11, **characterized in that** the base part (27) comprises a hook band, that the cuff (6) at least sectionally comprises a loop band, and that the hook band and the loop band together form a hook-and-loop connection (25) by means of which the base part (27) can be attached to the cuff (6) and be positioned at the same.

13. Orthosis according to claim 11 or 12 **characterized in that** the points at which one rope (2, 29) of a fastening device (26, 36) is held each are arranged on the base part (27) in pairs with the points at which the other rope (28, 29) of this fastening device (26, 36) is held.

14. Orthosis according to any of claims 12 to 13, **characterized in that** proceeding from one of the vertically spaced points each rope (28, 29) of at least one fastening device (26, 36) is guided through the base part (27), is thereby deflected in the base part (27) by an angle between 80° and 100°, and for tensioning the respective belt device (3, 4) is guided out of the base part (27) with one end of the rope (28, 29), and that the respective fastening device (26, 36) comprises a gripping part (32) associated to the base part (27), which is connected to the ends guided out of the ropes (28, 29) of the respective fastening device (26, 36).

## Revendications

1. Orthèse, notamment orthèse de gonarthrose destinée à décharger le compartiment médial ou latéral du genou humain, dotée d'un système de sangles (2), ledit système de sangles (2) présentant au moins un dispositif de sangles (3, 4) résistant à la traction, chaque dispositif de sangles (3, 4) présentant chacun au moins une sangle d'extrémité (9, 10, 11, 12), chaque dispositif de sangles (3, 4) présentant chacun au moins une sangle de liaison (7, 8) reliée à angle ajustable à chaque courroie d'extrémité (9, 10, 11, 12) du dispositif de sangles (3, 4) respectif,
l'orthèse (1) présentant un manchon (6) auquel est fixé le système de sangles (2), que ledit au moins un dispositif de sangles (3, 4) est constitué d'exactement un premier dispositif de sangles (3) doté d'une première sangle de liaison (7) et d'exactement un deuxième dispositif de sangles (4) doté d'une deuxième sangle de liaison (8), que les deux dispositifs de sangles (3, 4) se croisent l'un l'autre et sont chacun fixés à une zone proximale (33) et à une zone distale (34) du manchon (6),
**caractérisée en ce**
**que** le dispositif de sangles (2) présente des dispositifs de fixation (26, 36) dotés de cordelettes (28, 29) qui permettent de fixer les deux dispositifs de sangles (3, 4) au manchon (6), et que le système de sangles (2) présente des œillets (30, 31) qui permettent de maintenir les sangles d'extrémité (9, 10, 11, 12) des dispositifs de sangles (3, 4), chaque cordelette (28, 29) de chaque dispositif de fixation (26, 36) saillant au travers d'un œillet (30, 31) et maintenant ledit œillet (30, 31) au dispositif de fixation (26, 36) respectif dans un degré de liberté de translation et deux degrés de liberté de rotation.

2. Orthèse selon la revendication 1, **caractérisée en ce que** le système de sangles (2) présente un guide de croisement (5) pour deux dispositifs de sangles (3, 4), lesdits deux dispositifs de sangles (3, 4) se croisant au niveau dudit guide de croisement (5) et étant maintenus de manière forcée par ledit guide de croisement (5).

3. Orthèse selon la revendication 1 ou 2, **caractérisée en ce que** ladite au moins une sangle d'extrémité (9, 10, 11, 12) d'au moins un dispositif de sangles (3, 4) est constituée respectivement d'exactement une sangle d'extrémité proximale (9, 11) du dispositif de sangles (3, 4) respectif et d'exactement une sangle d'extrémité distale (10, 12) du dispositif de sangles (3, 4) respectif, et que la sangle de liaison (7, 8) du dispositif de sangles (3, 4) respectif présente une zone d'extrémité (13, 15) proximale permettant de relier la sangle de liaison (7, 8) respective à la sangle d'extrémité (9, 11) proximale du dispositif de sangles (3, 4) respectif, et présente une zone d'extrémité (14, 16) distale permettant de relier la sangle de liaison (7, 8) respective à la sangle d'extrémité (10, 12) distale du dispositif de sangles (3, 4) respectif.

4. Orthèse selon l'une quelconque des revendications 1 à 3, **caractérisée en ce qu'**au moins une sangle d'extrémité (9, 10, 11, 12) d'au moins un dispositif de sangles (3, 4) est reliée, à angle ajustable et de manière détachable au moyen d'une fermeture Velcro (18, 19, 21, 22), à la sangle de liaison du dispositif de sangles (3, 4) respectif.

5. Orthèse selon la revendication 4, **caractérisée en ce que** la sangle de liaison (7, 8) du dispositif de sangles (3, 4) respectif présente un élément de sangle doté d'une face recto et d'une face verso et présente, à chaque extrémité de l'élément de sangle (17, 20), sur sa face verso, respectivement une bande à crochets pour la fermeture Velcro (18, 19, 21, 22) et une languette agencée de manière correspondante à ladite bande à crochets et repliable, dotée d'une autre bande à crochets tournée vers ladite bande à crochets, que les sangles d'extrémité (9, 10, 11, 12) présentent de part et d'autre, au moins partiellement, une bande à boucle susceptible d'être positionnée entre les bandes à crochets, et que les sangles d'extrémité (9, 10, 11, 12) sont susceptibles d'être reliées à angle ajustable à la sangle de liaison (7, 8) au moyen de la fermeture Velcro (18, 19, 21, 22) réalisée par les bandes à crochets et les bandes à boucle.

6. Orthèse selon l'une quelconque des revendications 1 à 5, **caractérisée en ce qu'**au moins une sangle d'extrémité (9, 10, 11, 12) d'au moins un dispositif de sangles (3, 4) est reliée à angle ajustable à la sangle de liaison (7, 8) respective du dispositif de sangles (3, 4) respectif au moyen d'une liaison à vis ou au moyen d'une liaison à rivets.

7. Orthèse selon l'une quelconque des revendications 1 à 6, **caractérisée en ce qu'**au moins une sangle d'extrémité (9, 10, 11, 12) d'au moins un dispositif de sangles (3, 4) présente une portion de sangle inférieure (37) et une portion de sangle supérieure (38) rabattue sur la portion de sangle inférieure pour régler la longueur, lesdites portions de sangle (37, 38) formant une boucle réglable en longueur.

8. Orthèse selon la revendication 7, **caractérisée en ce que** le dispositif de sangles (3, 4) présente, au niveau de la portion de sangle supérieure (38) de la sangle d'extrémité (9, 10, 11, 12), une attache rapide (40) permettant de fermer la boucle de manière détachable, de préférence ladite attache rapide (40) présentant un aimant qui attire par magnétisme l'attache rapide (40) dans une position de fermeture et la verrouille dans ladite position de fermeture.

9. Orthèse selon la revendication 7 ou 8, **caractérisée en ce qu'**une extrémité de la sangle d'extrémité (9, 10, 11, 12) agencée sur la portion de courroie supérieure (38) est fixée au moyen d'une fermeture Velcro (39) dans une zone à sélectionner pour le réglage en longueur de la boucle.

10. Orthèse selon l'une quelconque des revendications 1 à 9, **caractérisée en ce qu'**au moins un dispositif de fixation (26, 36) est fixé sur le manchon (6) de manière à pouvoir être décalé verticalement.

11. Orthèse selon l'une quelconque des revendications 1 à 10, **caractérisée en ce qu'**au moins un dispositif de fixation (26, 36) présente une partie de base (27) sur laquelle chaque cordelette (28, 29) du dispositif de fixation (26, 36) respectif est maintenue à respectivement deux points espacés verticalement l'un de l'autre et forme respectivement une boucle avec la partie de base (27).

12. Orthèse selon la revendication 11, **caractérisée en ce que** la partie de base (27) présente une bande à crochets, que le manchon (6) présente au moins partiellement une bande à boucle et qu'ensemble, la bande à crochets et la bande à boucle constituent une fermeture Velcro (25) permettant de fixer et de positionner la partie de base (27) sur le manchon (6).

13. Orthèse selon la revendication 11 ou 12, **caractérisée en ce que** les points auxquels est maintenue une cordelette (2, 29) d'un dispositif de fixation (26, 36) sont agencés sur la partie de base (27), respectivement par deux avec les points auxquels l'autre cordelette (28, 29) dudit dispositif de fixation (26, 36) est maintenue.

14. Orthèse selon l'une quelconque des revendications 12 à 13, **caractérisée en ce que** chaque cordelette (28, 29) d'au moins un dispositif de fixation (26, 36) est guidée, à partir de respectivement un des points espacés verticalement les uns des autres, au travers de ladite partie de base (27), est déviée à cet égard dans la partie de base (27) dans un angle de 80° à 100° et dont une extrémité de la cordelette (28, 29) est conduite hors de la partie de base (27) pour tendre le dispositif de sangles (3, 4) respectif, et que le dispositif de fixation (26, 36) respectif présente une partie de préhension (32) associée à la partie de base (27), laquelle partie de préhension est reliée aux extrémités ressorties des cordelettes (28, 29) du dispositif de fixation (26, 36) respectif.
